# DEMANDE DE BREVET EUROPEEN

(11) **EP 1 161 938 A1**
(43) Date de publication de la demande: **12.12.2001**
(21) Numéro de dépôt: 01401198.5
(22) Date de dépôt: 10.05.2001
(51) Int. Cl.: A61K 7/48, A61K 7/00

(54) **Utilisation du phytantriol comme agent anti-pollution dans une composition cosmétique**

(30) Priorité: 08.06.2000 FR 0007343
(71) Demandeur: L'OREAL, 75008 Paris (FR)
(72) Inventeur: Biatry, Bruno, 94300 Vincennes (FR)
(74) Mandataire: Rasson, Catherine

(57) **Abrégé**

La présente demande se rapporte à l'utilisation en application topique, du phytantriol comme agent anti-pollution, notamment comme agent cosmétique anti-pollution.

La demande se rapporte aussi à un procédé de traitement cosmétique en vue de protéger l'organisme contre les effets de la pollution, consistant à appliquer sur la matière kératinique, une composition contenant dans un milieu physiologiquement acceptable, une quantité efficace de phytantriol.

Le phytantriol (3,7,11,15-tétraméthyl-1,2,3-hexadecanetriol) est de préférence dans une émulsion H/E ou E/H, ou sous forme de particules de gel cubique.

## Description

La présente demande se rapporte à l'utilisation en application topique, du phytantriol comme agent anti-pollution, notamment comme agent cosmétique anti-pollution, et à un procédé de traitement cosmétique en vue de protéger l'organisme contre les effets de la pollution, consistant à appliquer sur la matière kératinique, une composition contenant dans un milieu physiologiquement acceptable, une quantité efficace de phytantriol.

Certains milieux urbains sont régulièrement soumis à des pics de pollution. L'individu dans son environnement quotidien et particulièrement en zone urbaine, peut être soumis à de multiples agressions au niveau des matières kératiniques, et notamment de la peau, du cuir chevelu et des cheveux, par différents polluants aériens.

Parmi les polluants pouvant exercer des effets délétères sur les matières kératiniques, les gaz toxiques tels que l'ozone, le monoxyde de carbone, les oxydes d'azote ou les oxydes de soufre, sont l'un des constituants majeurs. Il a été constaté que ces gaz toxiques favorisent la desquamation des matières kératiniques, les rendent sales et ternes. De même, ces gaz entraînent une asphyxie cellulaire au niveau desdites matières kératiniques.

On sait par ailleurs que les métaux lourds (plomb, cadmium, mercure) sont des polluants atmosphériques dont les émissions ont notablement augmenté, notamment en milieu urbain ou industriel. Outre certains effets toxiques qui leur sont propres, les métaux lourds ont la propriété de diminuer l'activité des moyens de défense cellulaire contre les radicaux libres [voir par exemple R.S. Dwivedi, J. Toxicol. Cut. & Ocular Toxical. 6(3), 183-191 (1987)]. Ainsi, les métaux lourds aggravent les effets toxiques des polluants gazeux en diminuant l'efficacité des moyens naturels de défense, et provoquent une accélération du phénomène de vieillissement cellulaire. Ceci est vrai en particulier pour les matières kératiniques et notamment la peau, le cuir chevelu et les cheveux qui sont en contact direct et permanent avec le milieu extérieur.

Ainsi, les effets nocifs de la pollution sur les matières kératiniques affectent la respiration cellulaire et se traduisent par un vieillissement accéléré de la peau, avec un teint terne et la formation précoce de rides ou ridules, et aussi par une diminution de la vigueur des cheveux qui prennent aussi un aspect terne. On observe aussi comme effets nocifs de la pollution, la déshydratation de la peau. En outre, la pollution provoque une augmentation du flux du sébum, ayant pour conséquence que peau et cheveux se salissent plus rapidement. De plus, la pollution peut provoquer des phénomènes d'allergie et d'irritations sur la peau.

Ainsi, il existe le besoin de compositions permettant d'éviter les effets néfastes dus aux polluants (gaz ou métaux lourds), de façon à protéger les matières kératiniques de ces polluants externes.

Il a été maintenant constaté de manière tout à fait surprenante que le phytantriol permettait de protéger les matières kératiniques des effets des polluants se trouvant dans l'atmosphère.

Le phytantriol ou 3,7,11,15-tétraméthyl-1,2,3-hexadecanetriol est un composé connu, notamment commercialisé sous la dénomination de « Phytantriol-63926 » par la Société Roche.

Certes, l'utilisation du phytantriol en application topique est connue pour le soin de la peau (voir les documents GB-A-923400 et EP-A-0 584 315), comme agent hydratant (voir les documents GB-A-2,304,573 et EP-A-0 343 444), comme modulateur de pénétration (voir les documents EP-A-0 579 079 et GB-A-2,304,573). On connaît également son utilisation dans le domaine capillaire (voir les documents GB-A-944834, US-A-5,776,443 et WO-A-00/15181) et dans le maquillage (voir les documents FR-A-2,675,045 et US-A-5,102,654). Toutefois, aucun document ne décrit que le phytantriol puisse avoir des propriétés de protection des matières kératiniques contre la pollution.

Ainsi, la présente invention a pour objet l'utilisation cosmétique du phytantriol comme agent anti-pollution, dans une composition pour application topique sur les matières kératiniques.

L'invention a aussi pour objet l'utilisation du phytantriol pour la préparation d'une composition à application topique destinée à protéger la matière kératinique contre les effets nocifs de la pollution.

On entend ici par « application topique » une application externe sur les matières kératiniques, que sont notamment la peau, le cuir chevelu, les cils, les sourcils, les ongles et les muqueuses.

Le phytantriol peut être présent dans la composition pour application topique, en une quantité allant par exemple de 0,001 à 20 % en poids et de préférence de 0,1 à 10 % en poids par rapport au poids total de la composition.

Le phytantriol utilisé selon l'invention peut être incorporé notamment dans des compositions pouvant se présenter en particulier sous forme d'émulsions huile-dans-eau (H/E) ou eau-dans-huile (E/H) ou sous forme de particules de gel cubique, ces dernières pouvant être utilisées seules ou incorporées dans une émulsion.

Le terme de gel cubique désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans Luzzati (1968), « Biological Membranes » (Chapman, D. Ed.), vol 1, 71-123 et dans Mariani et coll. (1988), J. Mol. Biol., 204, 165-189, ainsi que dans "La Recherche" (1992), Vol.23, 306-315. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques. Tous les types de gel cubique peuvent être utilisés selon la présente invention.

Quand le gel cubique est dispersé dans un milieu aqueux, on obtient des particules de gel cubique en dispersion, particules qui ont la même structure que le gel cubique non dispersé.

Les particules de gel cubique contenant du phytantriol peuvent être présentes dans la composition pour application topique utilisée selon l'invention, en une quantité allant par exemple de 0,1 à 20 % en poids et de préférence de 0,1 à 10 % en poids par rapport au poids total de la composition.

De manière avantageuse, les particules de gel cubique contenant le phytantriol sont en dispersion aqueuse dans la composition pour application topique. Ces particules peuvent être notamment obtenues selon le mode préféré de réalisation décrit ci-dessous.

Selon ce mode de réalisation, les particules se trouvent en dispersion aqueuse et sont formées par un mélange comprenant (i) de 0,1 à 15 % en poids par rapport au poids total de la composition, de phytantriol ou d'un mélange de phytantriol avec un composé choisi parmi les dérivés N-2-alcoxycarbonyles de N-méthylglucamine et les monoglycérides d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone. Les pourcentages sont exprimés ici par rapport à la quantité totale de la composition contenant les particules de gel cubique à base de phytantriol.

Selon ce mode de réalisation des particules de gel cubique utilisées selon l'invention, la proportion pondérale relative en phytantriol par rapport au poids à l'agent dispersant et stabilisant (ii) peut aller par exemple de 2 à 200, et est de préférence inférieure ou égale à 50.

Parmi les dérivés N-2-alcoxycarbonyles de N-méthylglucamine pouvant être utilisés en mélange avec le phytantriol, on peut notamment citer ceux répondant à la formule (I) suivante : 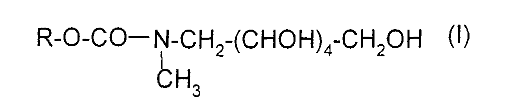 dans laquelle R représente un radical alkyle ramifié comportant de 6 à 18 atomes de carbone.

Parmi ceux-ci, on peut notamment mentionner la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

Les composés de formule (I) tels que définis ci-dessus sont décrits et peuvent être préparés selon le procédé décrit dans le document EP-A-711540 cité ici pour référence. Ce procédé comprend notamment les étapes consistant :
(a) à dissoudre dans un mélange d'eau et d'un solvant organique, de la N-méthyl-glucamine, le solvant pouvant être par exemple le tétrahydrofuranne,
(b) à disperser dans le mélange obtenu précédemment, du bicarbonate de sodium, en une proportion appropriée correspondant environ à quatre fois la proportion molaire de N-méthyl-glucamine,
(c) à introduire alors dans le mélange réactionnel obtenu, un chloroformiate d'alkyle, le radical alkyle étant en C6-C18, en une proportion appropriée, généralement équimolaire par rapport à celle de N-méthyl-glucamine, puis à laisser réagir le mélange, et
(d) à filtrer le mélange réactionnel obtenu après l'étape (c), à recueillir le résidu pâteux obtenu par filtration, puis à le dissoudre dans de l'acétone en vue de sa cristallisation à une température de l'ordre de 5°C. Après filtration, les cristaux du dérivé N-2-alcoxycarbonyle de N-méthylglucamine formé sont essorés et séchés sous vide.

Dans le cas où l'on utilise du phytantriol en mélange avec un ou des composés de formule (I), ce mélange comprend de préférence une quantité de phytantriol allant de 1 à 40 % en poids et mieux de 10 à 30 % en poids par rapport au poids du mélange, et une quantité de dérivé N-2-alcoxycarbonyle de N-méthylglucamine de formule (I) allant de 60 à 99 % en poids et mieux de 70 à 90 % en poids par rapport au poids du mélange.

Les monoglycérides d'acide gras insaturé pouvant être utilisés en mélange avec le phytantriol pour la préparation de particules de gel cubique sont de préférence ceux ayant une chaîne grasse insaturée comportant de 16 à 22 atomes de carbone. Parmi ceux-ci, on peut notamment citer le monooléate de glycéryle ou monooléine et le monolinoléate de glycéryle ou monolinoléine. On peut bien entendu utiliser, pour préparer les dispersions de particules de gel cubique, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Dans le cas où l'on utilise du phytantriol en mélange avec des monoglycérides d'acide gras insaturé, ce mélange comprend de préférence une quantité de phytantriol allant de 1 à 50 % en poids et mieux de 10 à 30 % en poids par rapport au poids total du mélange et une quantité de un monoglycéride d'acide gras insaturé en une proportion de 50 à 99 % en poids et mieux de 70 à 90 % en poids par rapport au poids du mélange.

L'agent dispersant et stabilisant (ii) des particules de gel cubique est de préférence choisi parmi :
(1) les alkyl ou alcényl éthers ou esters de polyol,
(2) les amino-acides N-acylés et leurs dérivés, et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de 8 à 22 atomes de carbone et peuvent se présenter sous forme de mélanges.
(1) Comme alkyl ou alcényl éthers ou esters de polyol, on peut citer notamment :
   (a) les alkyl ou alcényl esters de sorbitan polyoxyéthylénés par au moins 20 motifs d'oxyde d'éthylène tels que le palmitate de sorbitan 20 OE ou Polysorbate 40 commercialisé sous la dénomination de « Montanox 40 DF » par la Société Seppic, et le laurate de sorbitan 20 OE ou Polysorbate 20 commercialisé sous la dénomination de « Tween 20 » par la Société ICI,
   (b) les alkyl ou alcényl esters de polyglycérol comportant au moins 10 motifs dérivés du glycérol, oxyéthylénés ou non, tels que le polyglycéryl-10 laurate commercialisé sous la dénomination de « Decaglyn 1-L » par la Société Nikko Chemicals,
   (c) les alkyl ou alcényl éthers de polyglycérol, tels que le polyglycéryl-3 hydroxylauryléther commercialisé sous la dénomination de « Chimexane NF » par la Société Chimex, et
   (d) les alkyl ou alcényl éthers ou esters de mono ou polysaccharides, tels que ceux dérivant du glucose, du fructose, du galactose, du maltose ou du lactose et notamment les monoesters en 1- et 6- du D-fructose, du décylglucose et du décylpolyglucose.
(2) Comme amino-acides N-acylés et leurs dérivés, et les peptides N-acylés par un radical alkyle ou alcényle, et leurs sels, on utilise de préférence ceux pour lesquels le radical alkyle ou alcényle comporte au moins 12 atomes de carbone.
   Par amino-acides, on entend selon l'invention les alpha-, bêta- ou gamma-aminoacides. Comme sels d'amino-acides N-acylés, on peut citer par exemple ceux de N-acylglutamate, tels que le cocoylglutamate de monosodium, le lauroylglutamate de monosodium, l'alcoyl C14-C20 glutamate de disodium (le radical alcoyle C14-C20 dérivant du suif hydrogéné), commercialisés respectivement sous les dénominations de « Acylglutamate CS-11 », de « Acylglutamate LS-11 » et de « Acylglutamate HS-21 » par la Société Ajinomoto. On peut encore citer les lysines N-acylées telles que la lauroyl-lysine commercialisée sous la dénomination de « Amihope LL » par la Société Ajinomoto. Les dérivés d'amino-acides N-acylés et leurs sels sont de préférence les sarcosinates N-acylés tels que le lauroylsarcosinate de sodium commercialisé sous la dénomination de « Oramix L30 » par la Société Seppic, le myristoylsarcosinate de sodium et le palmitoylsarcosinate de sodium commercialisés respectivement sous les dénominations de « Nikkol Sarcosinate MN » et de « Nikkol Sarcosinate PN » par la Société Nikko Chemicals.
   Parmi les peptides N-acylés, on peut citer ceux dérivés de tout ou partie du collagène ou de la kératine, tels que le lauroyl collagène de sodium et la palmitoyl kératine commercialisés sous les dénominations de « Proteol B 30 » et de « Lipacide PK » par la Société Seppic.
(3) Parmi les alkyl ou alcényl éthers ou esters sulfates, leurs dérivés et leurs sels, on utilise de préférence ceux pour lesquels le radical alkyle ou alcényle comporte au moins 12 atomes de carbone.
   Parmi les alkyl ou alcényl éthers sulfates, on utilise de préférence les sels d'alkyl éther sulfate et notamment le lauryléther sulfate de sodium. Parmi les alkyl ou alcényl esters sulfates, on peut citer par exemple les esters de l'acide iséthionique ainsi que ses sels, et notamment le cocoyl iséthionate de sodium commercialisé sous la dénomination de « Geropon AC 78 » par la Société Rhône Poulenc.
(4) Parmi les alkyl ou alcényl éthers ou esters gras polyoxyéthylénés, on utilise de préférence ceux pour lesquels le radical alkyle ou alcényle comporte au moins 12 atomes de carbone. Ceux particulièrement préférés ont au moins 20 motifs d'oxyde d'éthylène, tels que par exemple le PEG-20 stéarate, le laureth-23, l'oleth-20 et le PEG-25 phytostérol.
(5) Parmi les acides alkyl ou alcényl carboxyliques polyoxyéthylénés et leurs sels, on utilise de préférence ceux comportant au moins 10 motifs d'oxyde d'éthylène, tels que par exemple l'acide laureth-10 carboxylique et l'acide oleth-10 carboxylique.
(6) Parmi les N-alkyl ou alcényl bétaïnes, on utilise de préférence celles pour lesquelles le radical alkyle ou alcényle comporte au moins 12 atomes de carbone telles que par exemple la lauryl-amidopropyl bétaïne et l'oléyl-amidopropyl bétaïne.
(7) Parmi les alkyl ou alcényl triméthylammonium et leurs sels, on utilise de préférence ceux pour lesquels le radical alkyle ou alcényle comporte au moins 12 atomes de carbone. Comme sels, on utilise de préférence les bromures et chlorures tels que le chlorure de cocoyltriméthylammonium et le bromure de cétyltriméthylammonium.

Selon un mode particulier de réalisation de l'invention, on peut ajouter dans la dispersion aqueuse contenant ces particules, un lipide amphiphile ionique non hydrosoluble, de préférence en une quantité allant de 0,0005 % à 5 % en poids et mieux de 0,001 % à 2 % en poids par rapport au poids total de la dispersion.

Parmi les lipides amphiphiles ioniques non hydrosolubles, on peut notamment citer :
(i) les phospholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoriques d'acide gras et leurs sels, notamment de sodium et de potassium, tels que le monocétyl phosphate commercialisé sous la dénomination de "Monafax 160" par la Société Mona, ainsi que le dimyristyl phosphate commercialisé sous la dénomination de « Mexoryl SY » par la Société Chimex,
(iii) les dérivés N-acylés de l'acide glutamique et leurs sels, tels que le stéaroylglutamate de monosodium commercialisé sous la dénomination de « Acylglutamate HS 11 » par la Société Ajinomoto, et le mélange cocoyl-alcoyl C14-C20 glutamate de monosodium, le radical alcoyle C14-C20 dérivant du suif hydrogéné, commercialisé sous la dénomination de « Acylglutamate GS 11 » par la Société Ajinomoto,
(iv) le cétylsulfate de sodium commercialisé sous la dénomination de « Nikkol SCS » par la Société Nikko Chemicals,
(v) le cocoyl monoglycéride sulfate de sodium commercialisé sous la dénomination de « Nikkol SGC 80 N » par la Société Nikko Chemicals, et
(vi) les dérivés d'ammonium quaternaire non hydrosolubles tels que le chlorure de béhényltriméthylammonium, le chlorure de dilauryldiméthylammonium, le chlorure de distéaryl-diméthylammonium, le méthylsulfate de 4,5-dihydro 1-méthyl 2-alcoyl C14-C20 1-(2-alcoyl C14-C20 aminoéthyl)imidazolium, les radicaux alcoyles C14-C20 dérivant du suif hydrogéné, commercialisé sous la dénomination de « Rewoquat W75H » par la Société Rewo Chemische, le méthylsulfate de dialkylhydroxyéthylméthylammonium dont les radicaux alkyles dérivent du suif, hydrogéné ou non, commercialisé sous la dénomination de « Stepanquat VP 85 » par la Société Stepan et le « Quaternium-82 » commercialisé par la Société Seppic sous la dénomination de « Amonyl DM ».

L'incorporation de ces lipides amphiphiles ioniques non hydrosolubles confère aux particules de gel cubique une charge superficielle entraînant une répulsion électrostatique des particules entre elles.

Les particules de gel cubique, telles que définies précédemment ont une taille qui peut être modulée par la nature et la concentration des composés les constituant. Généralement, ces particules ont une taille moyenne en nombre, mesurée à l'aide d'un granulomètre laser Bl 90 de la Société Brookhaven Instruments Corporation, d'environ 0,05 µm à environ 1 µm, et de préférence inférieure ou égale à 0,5 µm.

Il est en outre possible d'incorporer dans les particules de gel cubique, divers types de composés actifs. En particulier, lesdites particules peuvent contenir un principe actif hydrophile ou lipophile. Bien entendu, grâce à la structure particulière des particules de gel cubique, il est possible d'incorporer dans celles-ci à la fois des principes actifs hydrophiles et lipophiles, même si ceux-ci présentent une certaine incompatibilité.

De préférence, les dispersions de gel cubique contenant du phytantriol peuvent être obtenues selon un procédé de préparation comprenant au moins deux étapes. La première étape consiste généralement à préparer une dispersion aqueuse de particules de gel cubique telles que définies précédemment, par fragmentation, à l'aide d'un homogénéiseur, d'un gel cubique composé tel que défini précédemment et d'eau, en présence éventuellement de lipides amphiphiles ioniques non hydrosolubles et/ou de principes actifs hydrophiles et/ou lipophiles et/ou d'un agent dispersant et stabilisant tels que définis précédemment. L'homogénéiseur peut être du type rotor-stator à fort gradient de cisaillement comme le Virtis^{R} ou le Heidolph Diax 600^{R} ou un homogénéiseur haute pression fonctionnant entre 200 et 1.800 bars environ (20 à 180 MPa).

Il est bien entendu possible d'introduire, à ce stade de la préparation de la dispersion aqueuse des particules de gel cubique, divers additifs et/ou principes actifs dans la phase aqueuse. Après la formation des particules de gel cubique, l'agent dispersant et stabilisant se trouve, généralement, à l'extérieur desdites particules.

La deuxième étape consiste généralement ensuite à ajouter à ladite dispersion obtenue une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade de la préparation. Par ailleurs, lorsque l'on souhaite préparer une dispersion gélifiée, dans une troisième étape, on ajoute généralement au mélange obtenu après la seconde étape, une solution aqueuse contenant un agent gélifiant.

Les compositions contenant du phytantriol, utilisées selon l'invention peuvent constituer notamment des compositions cosmétiques et dermatologiques. Elles contiennent pour une telle application, un milieu physiologiquement acceptable. On entend ici par « milieu physiologiquement acceptable », un milieu compatible avec la peau, les lèvres, le cuir chevelu, les cils, les yeux et/ou les cheveux. Ce milieu physiologiquement acceptable peut être plus particulièrement constitué d'eau et éventuellement d'un solvant organique physiologiquement acceptable choisis par exemple parmi les alcools inférieurs comportant de 1 à 4 atomes de carbone comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Le milieu physiologiquement acceptable de la composition selon l'invention a un pH compatible avec la peau, et de préférence allant de 3 à 8 et mieux de 5 à 7.

Selon un mode préférée de réalisation, les compositions utilisées dans la présente invention comportent en outre une phase huileuse, apportant notamment du confort et de la douceur lors de l'application sur la peau. La quantité de phase huileuse peut aller par exemple de 2 à 40 % en poids et de préférence de 5 à 25 % en poids par rapport au poids total de la composition, le reste de la composition étant constitué de la phase aqueuse contenant ou constituée par la dispersion aqueuse des particules de gel cubique. Le rapport en poids des composés amphiphiles constituant les particules de la phase cubique et de la phase huileuse va de préférence de 0,02/1 à 1/1, et mieux de 0,05/1 à 0,5/1.

La phase huileuse comporte généralement au moins une huile. Comme huiles utilisables dans l'invention, on peut citer les huiles minérales (huile de vaseline, minéral oil), les huiles d'origine végétale (fraction liquide du beurre de karité, huile de tournesol, huile de ricin), les huiles d'origine animale (perhydrosqualène, huile de lanoline), les huiles de synthèse (polyisobutène hydrogénée, néopentanoate d'isostéaryle, myristate d'isopropyle), les huiles de silicone non volatiles ou volatiles (cyclométhicones telles que cyclopentasiloxane) et les huiles fluorées (perfluoropolyéthers). On peut aussi utiliser, comme matières grasses, des alcools gras, des acides gras, des cires. La phase huileuse de l'émulsion peut contenir aussi des gommes telles que les gommes de silicone, des résines et des cires.

La composition contenant une phase huileuse peut être sous forme d'une émulsion eau-dans-huile (E/H) ou huile-dans-eau (H/E). Selon un mode préféré de réalisation, elle est sous forme d'une émulsion huile-dans-eau.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 10 % du poids total de la composition. Ces adjuvants, selon leur nature, peuvent être introduits dans la phase grasse, dans la phase aqueuse et/ou dans des sphérules lipidiques.

Comme actifs, la composition peut notamment contenir d'autres actifs anti-pollution tels que les métallothionéines décrites dans le document EP-A-557 042, les sphingolipides (voir EP-A-0 577 718) et tout autre composé ayant la propriété d'éviter les effets néfastes des polluants ; des filtres tels que l'octocrylène et le butylméthoxydibenzoylméthane ; des hydratants tels que les polyols et notamment la glycérine.

Comme gélifiants, on peut citer par exemple les dérivés de cellulose tels que l'hydroxyéthylcellulose et les alkylhydroxyéthylcelluloses telles que la cétylhydroxyéthylcellulose ; les dérivés d'algues tels que le satiagum ; les gommes naturelles telles que l'adragante ou la gomme guar ; les polymères synthétiques tels que les polymères ou copolymères carboxyvinyliques et en particulier ceux commercialisés sous les dénominations de Carbopol^{R} par la société Goodrich ou de Synthalen^{R} par la société 3V SA. La proportion en agent gélifiant va de préférence de 0,1 à 2 % du poids total de la composition.

Les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Les compositions utilisées selon l'invention peuvent notamment constituer un produit de soin et/ou de maquillage. Elles peuvent être utilisées notamment pour protéger l'organisme et notamment les matières kératiniques, contre les effets de la pollution, et/ou pour améliorer la respiration cellulaire et/ou diminuer la desquamation des matières kératiniques, et notamment la peau, et/ou pour éviter l'augmentation du flux du sébum des matières kératiniques et donc pour éviter de ternir ou de salir les matières kératiniques, et notamment la peau. Elles peuvent être utilisées aussi pour éviter la déshydratation de la peau.

Ainsi, un autre objet de l'invention consiste en un procédé de traitement cosmétique en vue de protéger une matière kératinique contre les effets de la pollution, consistant à appliquer sur la matière kératinique, une composition contenant dans un milieu physiologiquement acceptable, une quantité efficace de phytantriol.

L'invention a aussi pour objet un procédé de traitement cosmétique en vue d'améliorer la respiration cellulaire et/ou diminuer la desquamation d'une matière kératinique et/ou pour éviter de ternir et/ou de salir une matière kératinique et/ou pour éviter la déshydratation d'une matière kératinique, consistant à appliquer sur la matière kératinique, une composition contenant dans un milieu physiologiquement acceptable, une quantité efficace de phytantriol.

Du fait que le phytantriol peut éviter la déshydratation de la peau, il est également approprié pour traiter les peaux sèches.

Aussi, l'invention a aussi pour objet un procédé de traitement cosmétique des peaux sèches, consistant à appliquer sur la peau, une composition contenant dans un milieu physiologiquement acceptable, une quantité efficace de phytantriol.

On entend par « quantité efficace » une quantité suffisante pour atteindre le but recherché. Dans la pratique, cette quantité peut aller par exemple, comme indiqué ci-dessus, de 0,001 à 20 % en poids et de préférence de 0,1 à 10 % en poids par rapport au poids total de la composition.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 :

### Phase A :

- Phytantriol 3,92 %
- Cetyl phosphate (commercialisé sous la dénomination de « Arlatone MAP160 » par la Société Uniqema) 0,08 %
- Eau 1,6 %

### Phase B :

- Polysorbate 40 (commercialisé sous la dénomination de « Montanox 40 DF » par la Société Seppic) (agent dispersant) 1 %
- Triéthanolamine 0,04 %
- Eau 55,96 %
- Conservateur 0,3 %

### Phase C :

- Polyisobutène hydrogéné 7,8 %
- Cyclohexasiloxane 11,6 %
- Néopentanoate d'isostearyle 2,6 %
- parfum 0,1 %

### Phase D :

- Cetyl hydroxyethylcellulose (commercialisé sous la dénomination de « Natrosol Plus Grade 330CS » par la Société Hercules) 1 %
- Eau 14 %

### Mode opératoire :

### Première étape :

On mélange à température ambiante les composés de la phase A. A ce mélange on ajoute à température ambiante la phase B. Le mélange est ensuite dispersé et homogénéisé à température ambiante à l'aide d'un homogénéiseur de type « UltraTurrax T50 » muni d'une tête de dispersion 45F, à 10000 rpm pendant 30 minutes.

### Deuxième étape :

A la dispersion aqueuse de phytantriol, obtenue ci-dessus, on ajoute le mélange huileux de la phase C. Le mélange est alors homogénéisé à température ambiante à l'aide d'un homogénéiseur haute pression par 4 passages à 600 bars.

### Troisième étape :

La préparation obtenue à la deuxième étape est gélifiée à l'aide du mélange de la phase D. On homogénéise alors le mélange à température ambiante à l'aide d'un homogénéiseur à pale pendant 30 minutes. On obtient une crème homogène et stable qui s'applique facilement sur la peau et la protège de la pollution.

### Exemple A (comparatif) : Emulsion huile dans eau

- Polyisobutène hydrogéné 7,8 %
- Cyclohexasiloxane 11,6 %
- Néopentanoate d'isostearyle 2,6 %
- Conservateurs 0,5 %
- Gomme de xanthane 0,6 %
- Polyacrylamide / C13-14 isoparaffin / laureth-7 (Sepigel 305 commercialisé par la société SEPPIC) 2 %
- Diméthicone copolyol (DC2-5695, Dow Corning) 3 %
- Glycérine 3 %
- Eau qsp 100 %

### Exemple B (comparatif) : Emulsion eau dans huile

- Polyisobutène hydrogéné 7,8 %
- Cyclohexasiloxane 11,7 %
- Néopentanoate d'isostearyle 2,6 %
- Chlorure de sodium 0,6 %
- Cétyldiméthicone copolyol (Abil EM90, Goldschmidt) 3 %
- Glycérine 3 %
- Eau qsp 100 %

### Test pour la mise en évidence in vitro de l'effet protecteur du phytantriol Efficacité anti-pollution sur peau reconstruite

Les compositions de l'exemple 1 et des exemples comparatifs A et B ont été appliquées à la surface des épidermes de peau reconstruite (2 mg/cm²) et laissées à leur contact pendant 30 minutes à température ambiante. Des particules radio-marquées au carbone 14 ont été ensuite déposées sur les épidermes et laissées à leur contact pendant 2 heures dans le milieu de maintenance habituelles des épidermes. Puis les épidermes ont été retirés de leur milieu de maintenance et lavés plusieurs fois au tampon PBS (Phosphate Buffer Solution). Les lavages permettent de débarrasser les épidermes des particules faiblement adsorbées sans éliminer la composition initialement appliquée. Les taux de particules radio-marquées résiduels ont été ensuite évalués par la mesure de la radioactivité du carbone 14 additionné aux particules. Le tableau ci-dessous donne les résultats en pourcentage de particules résiduelles par rapport à la quantité de particules déposées

| | % par rapport à la quantité de particules déposées |
|---|---|
| Zone non traitée | 36,2 ± 2,83% |
| Zone traitée avec composition de l'exemple 1 | 3,3 ± 0,66 |
| Zone traitée avec émulsion de l'exemple A (comparatif) | 11,3 ± 0,65 |
| Zone traitée avec émulsion de l'exemple B (comparatif) | 11,2 ±2,10 |

Ces résultats montrent que les compositions contenant du phytantriol, utilisées selon l'invention permettent une meilleure protection de la peau contre les particules polluantes que les émulsions classiques, en limitant la pénétration des particules polluantes externes.

### Exemple 2 : Fluide (émulsion H/E)

Selon le même mode opératoire que pour l'exemple 1, on a préparé un fluide de jour sous forme d'une dispersion par mélange des parties suivantes :

### Phase A :

- Phytantriol 2,97 %
- Stéaroylglutamate de monosodium, commercialisé sous la dénomination Acylglutamate HS-11 par la Société Ajinomoto 0,03 %
- Eau 1,25%

### Phase B :

- Polysorbate 40 (commercialisé sous la dénomination de « Montanox 40 DF » par la Société Seppic) (agent dispersant) 0,75 %
- Glycérine 4 %
- Propylène glycol 4 %
- Eau 55,8 %
- Conservateur 0,2 %

### Phase C :

- Octocrylène 8,4 %
- Butyl méthoxydibenzoylméthane 3,6 %
- Dimethicone (DC200 Fluid 1.5 cSt) 4 %
- Néopentanoate d'isostearyle 2 %
- Myristate d'isopropyle 2 %

### Phase D :

- Conservateur 1 %
- Eau 10 %

On obtient une composition fluide qui peut être appliquée sous forme d'un spray et qui permet une bonne protection de la peau contre les polluants.

### Exemple 3 : Emulsion huile dans eau

### Phase A :

- PEG-20 Stearate 4,5 %
- Phytantriol 3 %
- Sel di-sodique de l'acide N-stearoyl glutamique 0,5 %
- Minerai oil 10,51 %
- Huile de ricin 2,09 %
- Huile de vaseline 1,94 %
- Myristate d'isopropyle 1,39 %
- Huile de lanoline 1,07 %

### Phase B :

- Eau 69 %
- Glycérine 5 %
- Conservateur 1 %

On obtient une composition fluide qui permet une bonne protection de la peau contre les particules polluantes.

## Revendications

1. Utilisation cosmétique du phytantriol comme agent anti-pollution, dans une composition pour application topique sur les matières kératiniques.

2. Utilisation du phytantriol pour la préparation d'une composition à application topique destinée à protéger la matière kératinique contre les effets nocifs de la pollution.

3. Utilisation selon la revendication 1 ou 2, **caractérisée par le fait que** le phytantriol est présent en une quantité allant de 0,001 à 20 % en poids et de préférence de 0,1 à 10 % en poids par rapport au poids total de la composition.

4. Utilisation selon l'une quelconque des revendications précédentes, **caractérisée par le fait que** la composition contenant le phytantriol est une émulsion.

5. Utilisation selon l'une quelconque des revendications 1 à 3, **caractérisée par le fait que** le phytantriol est sous forme de particules de gel cubique.

6. Utilisation selon la revendication précédente, **caractérisée par le fait que** les particules de gel cubique sont en dispersion aqueuse.

7. Utilisation selon la revendication précédente, **caractérisée par le fait que** les particules de gel cubique sont formées par un mélange comprenant :
(i) de 0,1 à 15 % en poids par rapport au poids total de la composition, de phytantriol ou d'un mélange de phytantriol avec un composé choisi parmi les dérivés N-2-alcoxycarbonyles de N-méthylglucamine et les monoglycérides d'acide gras insaturé, et
(ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

8. Utilisation selon la revendication précédente, **caractérisée par le fait que** la proportion pondérale relative en composé (i) par rapport au poids dudit agent dispersant et stabilisant (ii) va de 2 à 200.

9. Utilisation selon la revendication 7 ou 8, **caractérisée par le fait que** ledit dérivé N-2-alcoxycarbonyle de N-méthylglucamine répond à la formule (I) suivante : 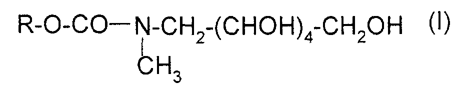 dans laquelle R représente un radical alkyle ramifié comportant de 6 à 18 atomes de carbone.

10. Utilisation selon l'une quelconque des revendications 7 à 9, **caractérisée par le fait que** ledit dérivé N-2-alcoxycarbonyle de N-méthyl-glucamine est choisi parmi la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

11. Utilisation selon l'une quelconque des revendications 7 à 10, **caractérisée par le fait que** les particules de gel cubique contiennent comme composé (i), un mélange constitué de 1 à 40 % en poids de phytantriol par rapport au poids du mélange et de 60 à 99 % en poids de dérivé N-2-alcoxycarbonyle de N-méthyl-glucamine par rapport au poids du mélange.

12. Utilisation selon la revendication 7, **caractérisée par le fait que** ledit monoglycéride d'acide gras insaturé est choisi parmi le monooléate de glycéryle et le monolinoléate de glycéryle.

13. Utilisation selon la revendication 7, **caractérisée par le fait que** les particules de gel cubique contiennent comme composé (i) un mélange constitué de 1 à 50 % en poids de phytantriol par rapport au poids du mélange, et de 50 à 99 % en poids de monoglycéride d'acide gras insaturé par rapport au poids du mélange.

14. Utilisation selon l'une quelconque des revendications 7 à 13, **caractérisée par le fait que** ledit agent dispersant et stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

15. Utilisation selon l'une quelconque des revendications 5 à 14, **caractérisée par le fait que** les particules de gel cubique ont une taille allant de 0,05 µm à 1 µm.

16. Utilisation selon l'une quelconque des revendications 6 à 15, **caractérisée par le fait que** la dispersion de particules de gel cubique comprend en outre au moins un lipide amphiphile ionique non hydrosoluble.

17. Utilisation selon la revendication précédente, **caractérisée par le fait que** ledit lipide amphiphile ionique non hydrosoluble est choisi parmi :
(i) les phospholipides,
(ii) les esters phosphoriques d'acide gras,
(iii) les dérivés N-acylés de l'acide glutamique non hydrosolubles et leurs sels,
(iv) le cétylsulfate de sodium,
(v) le cocoyl monoglycéride sulfate de sodium, et
(vi) les dérivés d'ammonium quaternaire non hydrosolubles.

18. Utilisation selon l'une quelconque des revendications 5 à 17, **caractérisée par le fait que** les particules comprennent au moins un principe actif hydrophile et/ou lipophile.

19. Utilisation selon l'une quelconque des revendications 5 à 17, **caractérisée par le fait que** les particules de gel cubique sont présentes en une quantité allant de 0,1 à 20 % en poids par rapport au poids total de la composition.

20. Procédé de traitement cosmétique en vue de protéger une matière kératinique contre les effets de la pollution, consistant à appliquer sur la matière kératinique, une composition comprenant dans un milieu physiologiquement acceptable, une quantité efficace de phytantriol.

21. Procédé de traitement cosmétique en vue d'améliorer la respiration cellulaire et/ou diminuer la desquamation d'une matière kératinique et/ou pour éviter de ternir et/ou de salir une matière kératinique et/ou pour éviter la déshydratation d'une matière kératinique, consistant à appliquer sur la matière kératinique, une composition comprenant dans un milieu physiologiquement acceptable, une quantité efficace de phytantriol.

22. Procédé de traitement cosmétique des peaux sèches, consistant à appliquer sur la peau, une composition comprenant dans un milieu physiologiquement acceptable, une quantité efficace de phytantriol.

23. Procédé selon l'une quelconque des revendications 20 à 22, **caractérisé par le fait que** la composition comprenant le phytantriol est une émulsion.

24. Procédé selon l'une quelconque des revendications 20 à 22, **caractérisé par le fait que** le phytantriol est sous forme de particules de gel cubique.

25. Procédé selon l'une quelconque des revendications 20 à 24, **caractérisé par le fait que** la matière kératinique est la peau.
